(19) Europäisches Patentamt
European Patent Office
Office européen des brevets

(11) Publication number: **0 100 651**
**A1**

# (12) EUROPEAN PATENT APPLICATION

(21) Application number: 83304351.6

(22) Date of filing: 27.07.83

(51) Int. Cl.³: A 61 K 7/42
C 07 C 69/708, C 07 C 69/618

(30) Priority: 03.08.82 US 404964
19.10.82 EP 82305565

(43) Date of publication of application:
15.02.84 Bulletin 84/7

(84) Designated Contracting States:
DE FR GB IT

(71) Applicant: VAN DYK & COMPANY, INC.
Main and William Streets
Belleville New Jersey 07109(US)

(72) Inventor: Conner, Donald E.
76 Washington Avenue
Clifton New Jersey 07011(US)

(72) Inventor: Cumpelik, Boris M.
41 Lincoln Terrace
Hillsdale New Jersey 07642(US)

(74) Representative: Eyles, Christopher Thomas et al,
BATCHELLOR, KIRK & EYLES 2 Pear Tree Court
Farringdon Road
London, EC1R 0DS(GB)

## (54) Dialkyl malonates as organic suncreen adjuvants.

(57) Compositions containing an organic sunscreen and substituted dialkyl malonate adjuvants therefor are very effective in providing broad spectrum sunscreen protection.

EP 0 100 651 A1

Croydon Printing Company Ltd.

DIALKYL MALONATES AS ORGANIC SUNSCREEN ADJUVANTS

Extensive studies have been made of the ultraviolet radiation of sunlight and skylight reaching the surface of the earth and the effects of such radiation on the human skin. It has been established that the radiation between 290 nanometres and 315 nanometres produces substantially all of the burning, or erythemal energy, and a substantial portion of the tanning energy, while the radiation between 315 nanometres and 400 nanometres promotes incident tanning. The cosmetic industry has divided these spectra into respectively UV-B, and UV-A. The different intensities and the erythemal and tanning effectiveness of the various wave lengths within these ranges have been established and methods have been determined for calculating accurately their effects on normal untanned skin.

Approximately 76% of the physiological tanning potential of sunlight is found in the ultraviolet range between 290 nanometres and 315 nanometres, the so-called UV-B or erythemal area; the balance is found in the range between 315 nanometres and 400 nanometres, the so-called UV-A or tanning area.

Typical organic sunscreens such as 2-ethylhexyl p-methoxy-cinnamate, homomenthyl salicylate, p-amino-benzoates, and hydroxy- and dihydroxy-4-methoxy-benzophenones, provide protection in the erythemal UV-B area, but lesser protection in the tanning area.

It is becoming increasingly apparent that ultraviolet in the tanning UV-A area can also have detrimental effects on skin health, e.g. causing premature ageing as well as skin cancer. Accordingly

the need has developed for more effective broad spectrum sun screens to filter out the entire radiation. This need can mean disrupting established formulation procedures, with the usual cosmetic oil carriers.

Some malonates have been disclosed as UV absorbers for industrial uses. Typically they are completely unsuitable for cosmetic purpose in human application, e.g. German Patent 1,087,902. A malonate, diethyl p-dimethyl-amino-benzalmalonate has been disclosed in U.S. Patent 3,895,104 as a conventional UV absorber in a polyamide resin film, but actually provides substantially no protection, even in the burning range.

It has now been found that certain dialkyl substituted malonates, i.e. dialkyl esters of certain substituted malonic acids, provide surprising protection in the tanning area, particularly around 370 nanometres, and are compatible with, and adjuvants for, organic sunscreens. This permits of utilizing established formulation procedures.

According to the present invention there is provided a composition adapted for application to the human skin comprising a cosmetic oil carrier containing distributed therein an organic sunscreen, such as 2-ethylhexyl p-methoxy-cinnamate, homomenthyl salicylate, p-aminobenzoic acid or one of its esters, p-dimethylaminobenzoic acid or one of its esters, a hydroxy- or dihydroxy-4-methoxybenzophenone, or a benzalphthalide, characterised in that it further comprises, as an adjuvant for the organic sunscreen, a substituted dialkyl malonate. Preferably the adjuvant is a substituted dialkyl malonate of the general formula:

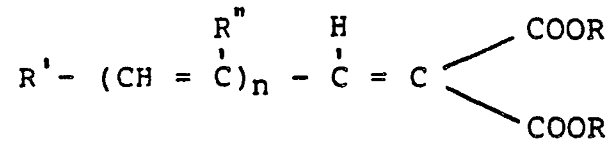 (I)

in which R' is an optionally substituted aromatic carbocyclic or heterocyclic group, n is 0 or 1, R" is a hydrogen atom or an alkyl group, and R is an alkyl group.

In another aspect the invention provides a method of protecting the human skin from the effects of erythema and tanning radiation in sunlight which comprises applying to said skin an organic sunscreen, such as 2-ethylhexyl p-methoxy-cinnamate, homomenthyl salicylate, p-amino-benzoic acid or one of its esters, p-dimethylamino benzoic acid or one of its esters, a hydroxy- or dihydroxy-4-methoxy-benzophenone, or a benzalphthalide, characterised in that there is further applied to said skin, as an adjuvant for the organic sunscreen, a substituted dialkyl malonate, preferably a substituted dialkyl malonate of the general formula (I) given above. Preferably the dialkyl malonate of the general formula (I) is selected from the group consisting of p-methoxy benzal diethyl malonate, p-methoxy benzal di-iso-butyl malonate, cinnamal diethyl malonate, indolal diethyl malonate, fural diethyl malonate, 3,4,5-trimethoxy benzal diethyl malonate, and diethyl α-methyl-cinnamal malonate.

The adjuvants useful in this invention are substituted dialkyl malonates in which the R alkyl group preferably has from 1 to 5 carbon atoms as illustrated in the following moiety:

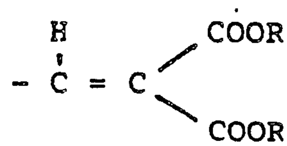

Typical groups R include methyl, ethyl, n-

and iso-propyl, n- and iso-butyl, and n- and iso-amyl groups.

As noted above n may be 0 or 1 in formula (I) whilst R" is a hydrogen atom or an alkyl group containing preferably from 1 to 4 carbon atoms, such as methyl or ethyl.

The efficacy of the dialkyl malonates as adjuvants are determined in an empiric manner. Thus the particularly preferred compounds are p-methoxy benzal diethyl malonate [diethyl 3-(4'-methoxyphenyl)-prop-1-en-1,1-dioate of the formula:

$p-CH_3O-C_6H_4-CH=C(COOC_2H_5)_2]$;

p-methoxy benzal di-iso-butyl malonate [di-i-butyl 3-(4'-methoxyphenyl)-prop-1-en-1,1-dioate) of the formula:

$p-CH_3O-C_6H_4-CH=C(COO-i-C_4H_9)_2$;

cinnamal diethyl malonate [diethyl 4-phenyl-butadien-1,1-dioate of the formula:

$C_6H_5-CH=CH-CH=C(COO-C_2H_5)_2]$;

indolal diethyl malonate (diethyl 3-(indol-2'-yl)-prop-1-en-1,1-dioate of the formula:

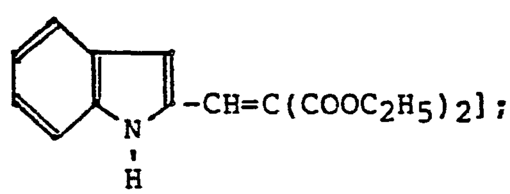

fural diethyl malonate [diethyl 3-(fur-2'-yl)-prop-1-en-1,1-dioate) of the formula:

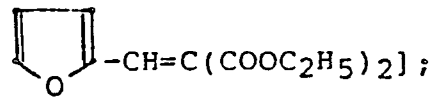

3,4,5-trimethoxy benzal diethyl malonate [diethyl 3-(3',4',5'-trimethoxyphenyl)-prop-1-en-1,1-dioate) of the formula:

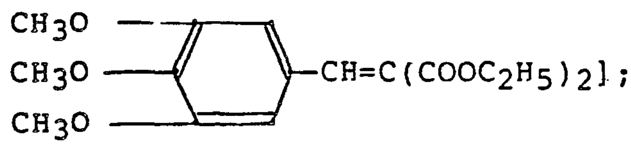

and diethyl α-methyl-cinnamal malonate [diethyl 4-phenyl-3-methyl-butadien-1,1-dioate of the formula:

$C_6H_5-CH=C(CH_3)-CH=C(COOC_2H_5)_2]$.

The cinnamal compound is especially effective.

The adjuvants of this invention can be prepared by a typical Knoevenagel reaction.

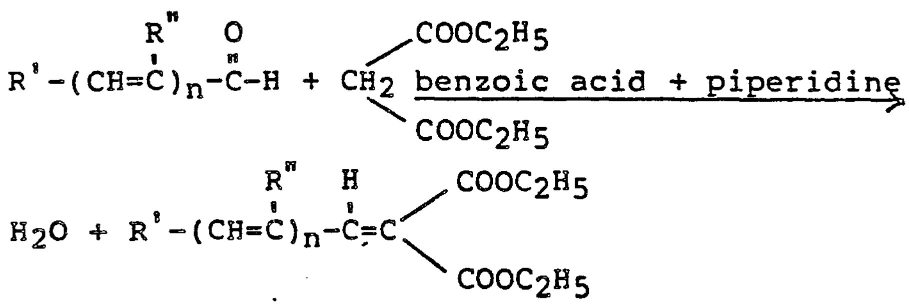


where R', n and R" are as defined above.

The solids can be crystallized from e.g. <u>iso</u>-propanol and the liquids can be fractionally distilled off under vacuum.

The organic sunscreens with which the adjuvants of this invention are employed are discussed above. A benzalphthalide may also be used, U.S. Patent 4,333,920, particularly 4-pentoxybenzalphthalide.

The overall composition adapted for

application to the human skin thus comprises a cosmetic oil carrier known to the trade, e.g. a carrier selected from mineral, vegetable and animal oils and iso-propyl myristate, with an organic sunscreen, and an adjuvant of this invention. The sunscreen and the adjuvant are utilized in an amount sufficient to provide the desired protection for the skin, more particularly in an effective amount to provide substantial protection against erythemal and tanning radiation. Typical total amounts of sunscreens and adjuvants comprise up to about 10 wt.% of the composition.

This invention, product workup and properties of the composition will be better understood by reference to the following examples.

Example 1

A mixture containing 35% 2,2-dihydroxy-4-methoxy benzophenone and 65% cinnamal diethyl malonate produced a total block when used in an otherwise conventional formulation based upon a cosmetic oil carrier at a concentration of between 5% and 10% in the final composition.

Example 2

Between 5%-6% of a mixture containing 50% 2-ethylhexyl-p-dimethylamino benzoate and 50% diethyl cinnamal malonate produced a total block when incorporated in an otherwise conventional formulation based upon a cosmetic oil carrier.

Example 3

Between 5%-5.5% of a mixture containing 50% 2-ethylhexyl-p-methoxy cinnamate and 50% diethyl cinnamal malonate produced a total block when incorporated in an otherwise conventional formulation based upon a cosmetic oil carrier.

Example 4

Between 4.5%-5% of a mixture containing 25% 4-pentoxy benzalphthalide and 75% diethyl p-methoxy benzal malonate produced a total block in an otherwise conventional formulation based upon a cosmetic oil carrier.

Example 5

Between 5%-6% of a mixture containing 30% di-iso-butyl p-methoxy benzal malonate and 70% 4-pentoxy benzalphthalide produced a total block when incorporated into an otherwise conventional formulation based upon a cosmetic oil carrier.

Example 6

Between 3%-4% of a mixture containing 50% diethyl fural malonate produced a total block when incorporated in a formulation that was based upon a cosmetic oil carrier and that was otherwise conventional.

Other formulations according to this invention provide similar results.

Substituted derivatives of the adjuvants of this invention can be employed, particularly of the cinnamal diethyl malonate. Such substituted derivatives include, for example, diethyl α-methyl-cinnamal malonate.

These examples demonstrate that the materials of this invention in even small quantities are extremely effective adjuvants for organic sunscreens and remedy the shortcomings of the latter.

As can be seen, mixtures of the materials of this invention, and also the organic sunscreens, can be employed where desired.

The advantages of this invention will be apparent to the skilled in the art. Improved, highly effective, novel broad spectrum sunscreen

compositions are made available, utilizing a dialkyl malonate.

It will be understood that this invention is not limited to the specific examples which have been offered as particular embodiments, and that modifications can be made without departing from the spirit thereof.

CLAIMS:

1. A composition adapted for application to the human skin comprising a cosmetic oil carrier containing distributed therein an organic sunscreen such as 2-ethylhexyl p-methoxy cinnamate, homomenthyl salicylate, p-aminobenzoic acid or one of its esters, p-dimethylaminobenzoic acid or one of its esters, hydroxy- or dihydroxy-4-methoxybenzophenone, or a benzalphthalide, characterised in that it further comprises, as an adjuvant for the organic sunscreen, a substituted dialkyl malonate.

2. A composition according to claim 1, characterised in that the adjuvant is a substituted dialkyl malonate of the general formula:

$$R' - (CH = \overset{R''}{\overset{|}{C}})_n - \overset{H}{\overset{|}{C}} = C\begin{matrix} \diagup COOR \\ \diagdown COOR \end{matrix} \qquad (I)$$

in which $R'$ is an optionally substituted aromatic carbocyclic or heterocyclic group, n is 0 or 1, $R''$ is a hydrogen atom or an alkyl group, and R is an alkyl group.

3. A composition according to claim 1 or claim 2, characterised in that the substituted dialkyl malonate is selected from the group consisting of p-methoxy benzal diethyl malonate, p-methoxy benzal di-iso-butyl malonate, cinnamal diethyl malonate, indolal diethyl malonate, fural diethyl malonate, 3,4,5-trimethoxy benzal diethyl malonate, and diethyl α-methyl-cinnamal malonate.

4. A composition according to any one of claims 1 to 3, _characterised in that_ the adjuvant is _p_-methoxy benzal diisobutyl malonate, cinnamal diethyl malonate, or diethyl α-methyl-cinnamal malonate.

5. A composition according to any one of claims 1 to 4, _characterised in that_ the organic sunscreen is 4-pentoxybenzalphthalide.

6. A method of protecting the human skin from the effects of erythema and tanning radiation in sunlight which comprises applying to said skin an effective sunscreening amount of an organic sunscreen, such as 2-ethylhexyl _p_-methoxy cinnamate, homomenthyl salicylate, _p_-amino-benzoic acid or one of its esters, _p_-dimethylamino benzoic acid or one of its esters, hydroxy- or dihydroxy-4-methoxy-benzophenone, or a benzalphthalide, _characterised in that_ there is further applied to said skin, as an adjuvant for the organic sunscreen, a substituted dialkyl malonate.

7. A method according to claim 6, _characterised in that_ the adjuvant is a substituted dialkyl malonate of the general formula (I) given in claim 2.

8. A method according to claim 6 or claim 7, _characterised in that_ the substituted dialkyl malonate is selected from the group consisting of _p_-methoxy benzal diethyl malonate, _p_-methoxy benzal di-_iso_-butyl malonate, cinnamal diethyl malonate, indolal diethyl malonate, fural diethyl malonate, 3,4,5-tri-methoxy benzal diethyl malonate, and diethyl α-methyl-cinnamal malonate.

9. A method according to any one of claims 6 to 8, characterised in that the adjuvant is *p*-methoxy benzal di-*iso*-butyl malonate or cinnamal diethyl malonate, or diethyl α-methyl-cinnamal malonate.

10. A method according to any one of claims 6 to 9, characterised in that the organic sunscreen is 4-pentoxybenzalphthalide.

European Patent Office

# PARTIAL EUROPEAN SEARCH REPORT

which under Rule 45 of the European Patent Convention shall be considered, for the purposes of subsequent proceedings, as the European search report

Application number
EP 83 30 4351

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X,Y | GB - A - 1 064 116 (ASPRO NICHOLAS)<br>* Claims 1,21-27; page 4, lines 15-32; examples 8,9 * | 1,2,6,7 | A 61 K 7/42<br>C 07 C 69/708<br>C 07 C 69/618 |
| | -- | | |
| X,Y | FR - A - 2 219 930 (BAYER)<br>* Claims 1-4; page 4, line 35 - page 5, line 13 * | 1,2 | |
| | -- | | |
| Y | GB - A - 1 037 169 (AMERICAN CYANAMID)<br>* Claims 12,16,17 * | 1-3 | |
| | -- | | |
| Y | US - A - 3 125 597 (WAHL et al.)<br>./. | | |

TECHNICAL FIELDS SEARCHED (Int. Cl. 3)

A 61 K 7/00
C 08 K 5/00
G 03 C 1/00
C 07 C 69/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:
Claims searched incompletely:
Claims not searched: 6-10
Reason for the limitation of the search:

Method for treatment of the human or animal body by surgery or therapy (see article 52 (4) of the European Patent Convention).

| Place of search | Date of completion of the search | Examiner |
|---|---|---|
| The Hague | 06-09-1983 | WILLEKENS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03 82

European Patent Office

# PARTIAL EUROPEAN SEARCH REPORT

Application number

-2- EP 83 30 435

| DOCUMENTS CONSIDERED TO BE RELEVANT | | | CLASSIFICATION OF THE APPLICATION (Int Cl.3) |
|---|---|---|---|
| Category | Citation of document with indication, where appropriate, of relevant passages | Relevant to claim | |
| | * Column 1, lines 10-39; column 2, lines 55-60; example 6 * | 1-4 | |
| | -- | | |
| Y | US - A - 4 322 455 (OLSON) | | |
| | * Column 6, lines 6-40; example 3 * | 1-4 | |
| | -- | | |
| D,Y | US - A - 4 333 920 (CONNER) | | TECHNICAL FIELDS SEARCHED (Int. Cl.3) |
| | * Claims * | 1,5 | |
| | -- | | |
| A | DE - A - 2 410 714 (SHIONOGI & CO.) | | |
| | * Page 3, formula VI; page 6, lines 9-21 * | 3 | |
| | -- | | |
| A | BULLETIN DE LA SOCIETE CHIMIQUE DE FRANCE, no. 9/10, 10 September 1978, MASSON PARIS (FR) S. REBUFFAT et al.: "Synthèse stéréospécifique d'α- carboxy-esters éthyléniques par extension de la réaction de Stobbe", pages II-457-II-460. | | |
| | * Page 457, right-hand column, first formula; page 459, table; compound 1c * | 3,4 | |
| | ---- | | |